(19) Europäisches Patentamt
European Patent Office
Office européen des brevets

(11) Veröffentlichungsnummer : **0 516 660 B1**

(12) # EUROPÄISCHE PATENTSCHRIFT

(45) Veröffentlichungstag der Patentschrift :
**18.08.93 Patentblatt 93/33**

(51) Int. Cl.$^5$ : **A61K 31/70**

(21) Anmeldenummer : **91903897.6**

(22) Anmeldetag : **20.02.91**

(86) Internationale Anmeldenummer :
**PCT/EP91/00319**

(87) Internationale Veröffentlichungsnummer :
**WO 91/12809 05.09.91 Gazette 91/21**

(54) **VERWENDUNG VON S-ADENOSYLMETHIONIN ALS MITTEL GEGEN VIRALE INFEKTIONEN DURCH RETROVIREN.**

(30) Priorität : **20.02.90 DE 4005275**

(43) Veröffentlichungstag der Anmeldung :
**09.12.92 Patentblatt 92/50**

(45) Bekanntmachung des Hinweises auf die Patenterteilung :
**18.08.93 Patentblatt 93/33**

(84) Benannte Vertragsstaaten :
**AT BE CH DE DK FR GB IT LI LU NL SE**

(56) Entgegenhaltungen :
**J. of Medicinal Chemistry, vol. 23, no. 4, April 1980, Am. Chem. Soc., R.T. Borchardt, pp. 347-357**
**J. of Medicinal Chemistry, vol. 32, no. 7, 1989, Am. Chemical Soc., C.K.H. Tseng et al., pp. 1442-1446**
**The Merck Index, 11th ed., 1989, Merck & Co., Inc., (Rahway, N.J., US), p. 26, Monographie 146: "S-Adenosylmethionine"**

(73) Patentinhaber : **GRUNDMANN, Ewald**
**Pregelstrasse 26**
**D-45136 Essen (DE)**

(72) Erfinder : **GRUNDMANN, Ewald .Dr.**
**Pregelstrasse 26**
**W-4300 Essen 1 (DE)**
Erfinder : **RÜBSAMEN-WAIGMANN, Helga. Prof.Dr.**
**Königsteiner Strasse 113**
**W-6232 Bad Soden / Taunus (DE)**

(74) Vertreter : **Wolgast, Rudolf, Dipl.-Chem. Dr. et al**
**Dipl.-Phys. Jürgen Weisse Dipl.-Chem. Dr. Rudolf Wolgast Bökenbusch 41 Postfach 11 03 86**
**D-42531 Velbert (DE)**

EP 0 516 660 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die Erfindung betrifft die Verwendung von S-Adenosylmethionin zur Herstellung eines Arzneimittels gegen virale Infektionen durch Retroviren, insbesondere durch HI (human immunodeficiency) Viren bzw. gegen AIDS-Infektionen.

S-Adenosylmethionin

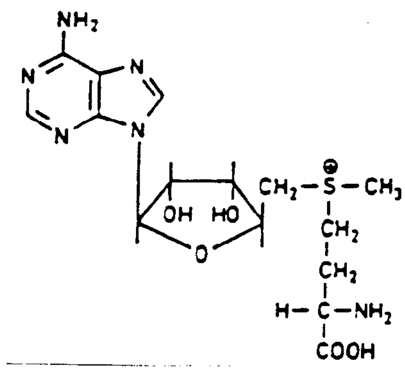

ist ein Sulfoniumsalz, das in biologischen Systemen weit verbreitet ist und im $C_1$-Stoffwechsel als Methylierungsagens, d.h. als Überträger von Methylgruppen auftritt. So ist beispielsweise aus der Veröffentlichung von F. Hirata und J. Axelrod unter dem Titel "Phospholipid Methylation and Biological Signal Transmission" (Science 209, S. 1082 bis 1090; 1980) bekannt, daß S-Adenosylmethionin in Zellmembranen die Methylierung von Phospholipiden bewirkt, wobei Phosphatidylethanolamin unter Mitwirkung von zwei Methyltransferasen in zwei Methylierungsschritten in Phosphatidylcholin umgewandelt wird, wodurch sich auch die Fluidität der Zellmembran von zum Beispiel Erythrocyten ändert.

Weiterhin ist aus einer Veröffentlichung von R. Holliday unter dem Titel "A Different Kind of Inheritance" (Scientific American, Juni 1989, S. 40 bis 48) bekannt, daß durch S-Adenosylmethionin in DNA (Desoxyribonukleinsäure) enthaltene Cytosingruppen in 5-Methylcytosingruppen umgewandelt werden, was zur Desaktivierung von Genen in der DNA führen kann.

Schließlich ist S-Adenosylmethionin, das als solches in Lösung unbeständig ist, in stabilisierter Form als Tosylat-bis-Sulfat gelöst und in oraler Form als Heilmittel für Gelenkerkrankungen zugelassen.

Die parenterale Applikation ist in der Bundesrepublik Deutschland zur Zulassung eingereicht worden. Bei intravenöser Applikation liegen die verträglichen Dosierungen in Bereich von 100 bis 500 mg (1,5 bis 7,5 mg/kg), vgl. die Veröffentlichung von F. Giulidori et al. unter dem Titel "Pharmacokinetics of S-Adenosyl-1-methionine in Healthy Volunterrs" in Eur. J. Clin. Pharmacol. (1984) 27; S. 119 bis 221.

Überraschenderweise wurde nun durch in-vitro Versuche festgestellt, daß S-Adenosylmethionin in stabilisierter Form trotz seiner hohen Reaktivität gegenüber den vielfältigsten Stoffwechselkomponenten in Konzentrationen im Bereich von 100 bis 200µg/ml eine Wirksamkeit gegen virale Infektionen durch Retroviren, insbesondere HIV oder AIDS-Viren zeigt. Das wird durch die nachfolgend aufgeführten Versuche belegt.

BEISPIEL

Lymphocyten-Kulturen aus der Nabelschnur von Neugeborenen (H. Rübsamen-Waigmann et al. "Isolation of Variants of Lymphocytopathic Retroviruses from the Peripheral Blood and Cerebrospinal Fluid of Patients with ABC or AIDS", J. Medical Virology 19, S. 335 bis 344; 1986) wurden mit Viren, die dem Blut von AIDS-Kranken entnommen waren, geimpft. Dazu werden zu 5 ml der Lymphocyten-Kultur ($1 \cdot 10^6$ Zellen pro ml) 0,5 ml einer HIV-Suspension gegeben, die $10^4$ bis $10^5$ infektiöse Partikel pro ml enthält. Die Kulturmedien werden mit unterschiedlichen Mengen S-Adenosyl-L-methionintosylat-bis-Sulfat versetzt. Ein Drittel bis zur Hälfte des jeweiligen Kulturmediums werden nach 1 bis 2 Tagen durch frisches Kulturmedium gleicher Zusammensetzung ersetzt.

Retroviren zeichnen sich dadurch aus, daß sie ausschließlich RNA (Ribonukleinsäure) als Nukleinsäure enthalten und eine spezielle Polymerase, nämlich Reverse Transkriptase benötigen, die nach der Infektion in der Wirtszelle aus der Virus-RNA eine in die DNA der Wirtszelle einbaubare Virus-DNA bildet, vgl. H. Rübsamen "Biochemie der Tumorviren", Chemie in unserer Zeit 17, S. 169 bis 180; 1983. Daraus ergibt sich eine Nachweismethode für Retroviren, die auf der für diese typischen Reversen Transkriptase und dem durch

diese in die DNA eingebauten Tritium-markierten Thymidin beruht, vgl. H. Rübsamen et al., l.c.:

In Abhängigkeit vom positiven Nachweis der Infektion durch mikroskopische Kontrolle werden nach 3 bis 5 Tagen jeweils 5 ml der überstehenden Kulturflüssigkeit durch Zentrifugieren (1o min bei 10.000 g) geklärt. Die so geklärte Probe wird bei 100.000 g/50 min zentrifugiert und der Rückstand in 60 µl eines Tris-Puffers lysiert (10 mM Tris, 0,2 % Triton X-100, 1 mM EDTA, 5 mM Dithiothreitol, 60 mM KCl, pH 7,3).

Jeweils 30 µl des Lysats werden mit 30 µl Templat (0,01 mg/ml Poly(rA)·oligo (dT) (Pharmacia, Uppsala, Schweden) in 5 mM $MgCl_2$, 2,4 mM Dithiothreitol, 60 mM KCl und 0,0034 mM Methyl-[$^3$H]thymidin-5'-Triphosphat; 48 Ci/mM, Amersham, Bucks; England) 90 min bei 37° C inkubiert. Die Reaktion wird durch Zugabe von 10-proz. Laurylsarkosin bis zu einer Laurylsarkosin-Endkonzentration von 1,4 % beendet. Die freie und in das RNA/DNA-Hybrid eingebaute Radioaktivität von 10 µl des mit Laurylsarkosin versetzten Inkubats werden dann in üblicher Weise an einem DEAE-Filter getrennt, das nach dem Waschen und Trocknen in eine Szintillationsflüssigkeit gegeben wird. Die Radioaktivität wird dann mit einem Szintillationszähler gemessen. Die eingebaute Radioaktivität (Zerfälle pro min pro 5 ml) nach Abzug des Blindwertes ist dann ein Maß für die in der überstehenden Kulturflüssigkeit enthaltene Reverse Transkriptase und damit für die Menge an Retroviren, die in der Kultur enthalten sind.

Die Versuchsergebnisse sind in der nachfolgenden Tabelle und in Form eines Diagramms dargestellt. Es ergibt sich daraus, daß die Zahl der Retroviren mit zunehmender Menge an S-Adenosyl-L-methionintosylat-bis-Sulfat im Kulturmedium abnimmt. Die Halbwertskonzentration, bei der die Retrovirenkonzentration auf die Hälfte zurückgeht, liegt im Bereich von 100 bis 200 µg/ml.

## Tabelle

### HIV-Zahl in Lymphocyten-Kultur als Funktion der Adenosylmethionin-Konzentration, bestimmt als radioaktive Reverse Transkriptase

| Adenosylmethionin (µg/ml) | Reverse Transkriptase (counts/min und 5 ml) |
|---|---|
| 4 | 114 000 |
| 40 | 86 000 |
| 200 | 73 000 |
| 400 | 60 000 |
| 1000 | 29 000 |

Die vorstehend beschriebene Wirkung von S-Adenosylmethionin kann verschiedene mögliche Ursachen haben. So könnte beispielsweise die Lymphocytenmembran durch Methylierung verändert und dadurch der Rezeptor für die Virusbindung beeinflußt werden. Es könnte aber auch eine Methylierung der Virus-RNA und/oder der daraus in den Lymphocyten gebildeten Virus-DNA erfolgen und dadurch die Replikation bzw. Virus-Synthese gehemmt werden; in diesem Falle müßte allerdings das S-Adenosylmethionin als intaktes Molekül die Virusmembran und/oder die Lymphocytenmembran passieren können. Diese möglichen Wirkungsmechanismen sind nicht mit dem des bekannten AIDS-Mittels AZT(3'-Azido-2',3'-didesoxythymidin) vergleichbar, das nach Phosphorylierung die Bindung von Thymidin an die Reverse Transkriptase kompetitiv hemmt.

**Patentansprüche**

1. Verwendung von S-Adenosylmethionin zur Herstellung eines Arzneimittels gegen virale Infektionen durch Retroviren.

2. Verwendung von S-Adenosylmethionin zur Herstellung eines Arzneimittels gegen HIV-Infektion.

3. Verwendung von S-Adenosylmethionin nach Anspruch 1 oder 2, dadurch gekennzeichnet, daß das S-Adenosylmethionin stabilisiert ist.

4. Verwendung von S-Adenosylmethionin nach Anspruch 3, dadurch gekennzeichnet, daß das S-Adenosylmethionin in form des bis-Sulfats als Tosylat stabilisiert ist.

## Claims

1. Method of using S-adenosyl methionine for the manufacture of a medicament against viral infections by retroviruses.

2. Method of using S-adenosyl methionine for the manufacture of a medicament against HIV infections.

3. Method of using S-adenosyl methionine according to claim 1 or 2, characterised in that the S-adenosyl methionine is stabilised.

4. Method of using S-adenosyl methionine according to claim 3, characterised in that the S-adenosyl methionine is stabilised in the form of the bis-sulfate as tosylate.

## Revendications

1. Utilisation de S-adénosine-méthionine destinée à la production d'un médicament contre des infections virales par des rétrovirus.

2. Utilisation de S-adénosine-méthionine destinée à la production d'un médicament contre l'infection HIV.

3. Utilisation de S-adénosine-méthionine selon la revendication 1 ou 2, caractérisée par le fait que la S-adénosine-méthionine est stabilisée.

4. Utilisation de S-adénosine-méthionine selon la revendication 3, caractérisée par le fait que la S-adénosine-méthionine est stabilisée en forme de bisulfate comme tosylate.

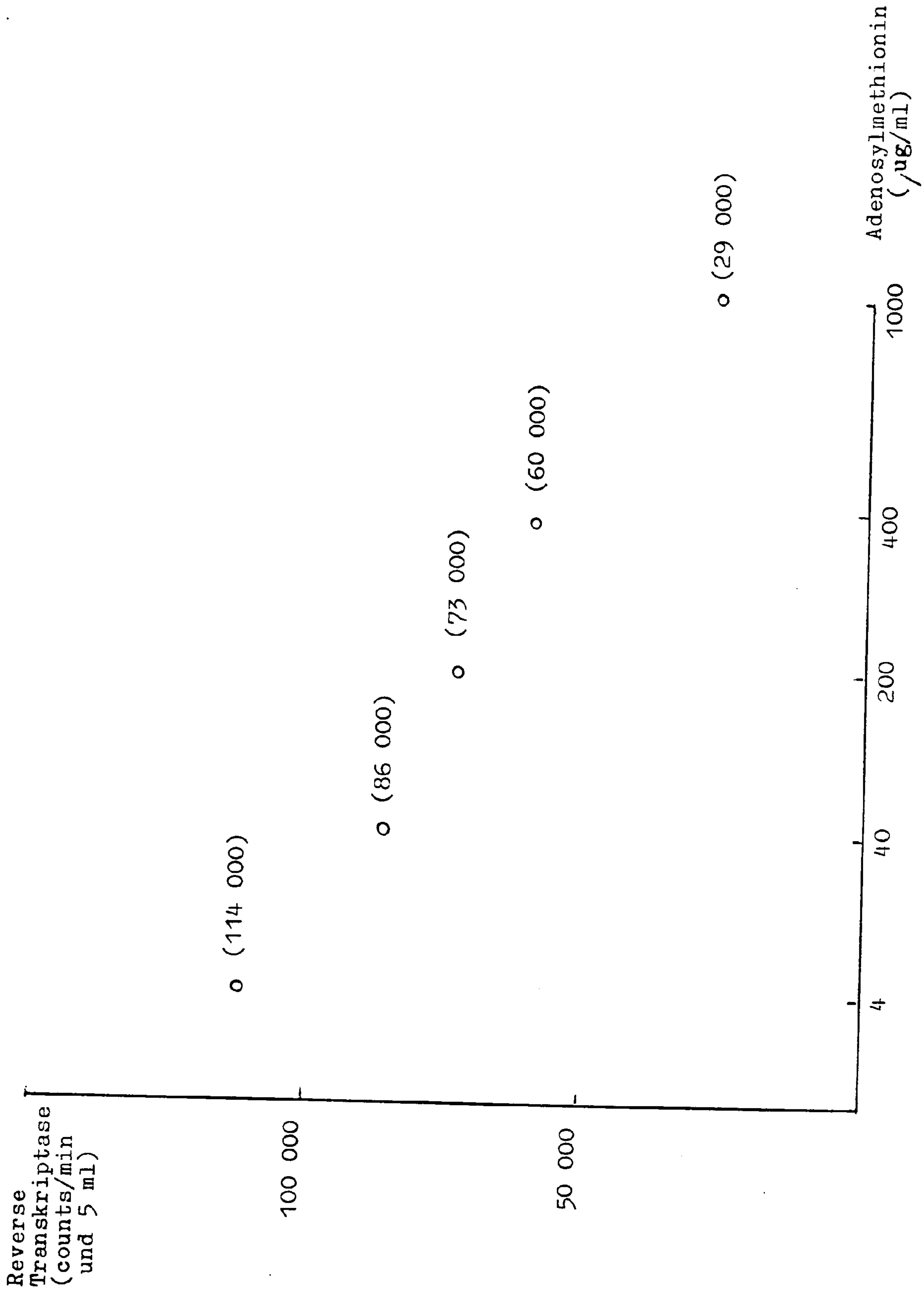
Reverse Transkriptase (counts/min und 5 ml)
100 000
50 000
Adenosylmethionin (/ug/ml)
4
40
200
400
1000
(114 000)
(86 000)
(73 000)
(60 000)
(29 000)